# EUROPEAN PATENT SPECIFICATION

(11) **EP 2 509 633 B1**
(45) Date of publication and mention of the grant of the patent: **18.06.2025**
(21) Application number: 10836583.4
(22) Date of filing: 08.12.2010
(51) Int. Cl.: A61K 9/51, A61K 33/20, A61P 13/00, A61P 17/00, A61P 19/02, A61P 19/04, A61P 27/02, A61P 29/00, A61P 35/00, A61K 41/00

(54) **NANOPARTICLE CARRIER SYSTEM BASED ON POLY(DL-LACTIC-CO-GLYCOLIC ACID) (PLGA) FOR PHOTODYNAMIC THERAPY**
NANOPARTIKEL-TRÄGERSYSTEM AUF BASIS VON POLY-(DL-MILCHSÄURE-CO-GLYKOLSÄURE) (PLGA) FÜR DIE PHOTODYNAMISCHE THERAPIE
SYSTÈME DE SUPPORT NANOPARTICULAIRE À BASE DE POLY(ACIDE LACTIQUE-CO-GLYCOLIQUE) (PLGA) POUR LA THÉRAPIE PHOTODYNAMIQUE

(30) Priority: 11.12.2009 US 285895 P; 08.11.2010 US 941447
(43) Date of publication of application: 17.10.2012
(73) Proprietor: biolitec Holding GmbH & Co KG, 1030 Wien (AT)
(72) Inventor: LANGER, Klaus, 48161 Münster (DE); KNOBLOCH, Thomas, 60385 Frankfurt (DE); RÖDER, Beate, 14612 Falkensee (DE); PREUSS, Annegret, 13189 Berlin (DE); ALBRECHT, Volker, 14558 Nuthtetal (DE); GRÄFE, Susanna, 07745 Jena (DE); WIEHE, Arno, 10777 Berlin (DE); von BRIESEN, Hagen, 65510 Hünstetten (DE); LÖW, Karin, 66740 Saarlouis (DE); WAGNER, Sylvia, 66539 Neunkirchen (DE)
(74) Representative: Herrmann, Franz
(86) International application number: PCT/US2010/059367
(87) International publication number: WO 2011/071970

(56) References cited:
- WO-A1-03/097096
- WO-A2-2006/133271
- US-A- 5 998 597
- US-A1- 2006 040 913
- VARGAS ANGELICA ET AL: "Toward the understanding of the photodynamic activity of m-THPP encapsulated in PLGA nanoparticles: correlation between nanoparticle properties and in vivo activity", JOURNAL OF DRUG TARGETING, vol. 17, no. 8, Sp. Iss. SI, September 2009 (2009-09-01), pages 599 - 609, XP009185401, ISSN: 1061-186X
- KONAN Y N ET AL: "ENCAPSULATION OF P-THPP INTO NANOPARTICLES: CELLULAR UPTAKE, SUBCELLULAR LOCALIZATION AND EFFECT OF SERUM ON PHOTODYNAMIC ACTIVITY", PHOTOCHEMISTRY AND PHOTOBIOLOGY, WILEY-BLACKWELL PUBLISHING, INC, US, vol. 77, no. 6, 2003, pages 638 - 644, XP008022464, ISSN: 0031-8655, DOI: 10.1562/0031-8655(2003)077<0638:EOPINC>2.0.CO;2
- KONAN Y N ET AL: "Preparation and characterization of sterile sub-200 nm meso-tetra(4-hydroxylphenyl)porphyrin-loaded nanoparticles for photodynamic therapy", EUROPEAN JOURNAL OF PHARMACEUTICS AND BIOPHARMACEUTICS, ELSEVIER SCIENCE PUBLISHERS B.V., AMSTERDAM, NL, vol. 55, no. 1, 2003, pages 115 - 124, XP004404966, ISSN: 0939-6411, DOI: 10.1016/S0939-6411(02)00128-5
- CHATTERJEE D K ET AL: "Nanoparticles in photodynamic therapy: An emerging paradigm", ADVANCED DRUG DELIVERY REVIEWS, ELSEVIER, AMSTERDAM, NL, vol. 60, no. 15, 14 December 2008 (2008-12-14), pages 1627 - 1637, XP025656957, ISSN: 0169-409X, [retrieved on 20080920], DOI: 10.1016/J.ADDR.2008.08.003
- RANCAN F ET AL: "Influence of substitutions on asymmetric dihydroxychlorins with regard to intracellular uptake, subcellular localization and photosensitization of Jurkat cells", JOURNAL OF PHOTOCHEMISTRY AND PHOTOBIOLOGY B: BIOLOGY, ELSEVIER SCIENCE S.A., BASEL, CH, vol. 78, no. 1, 14 January 2005 (2005-01-14), pages 17 - 28, XP027789457, ISSN: 1011-1344, [retrieved on 20050114]
- AL-OMARI S: "Photophysical properties and localization of chlorins substituted with methoxy groups, hydroxyl groups and alkyl chains in liposome-like cellular membrane; Photophysical properties and localization of chlorins in liposome-like cellular membrane", BIOMEDICAL MATERIALS, INSTITUTE OF PHYSICS PUBLISHING, BRISTOL, GB, vol. 2, no. 2, June 2007 (2007-06-01), pages 107 - 115, XP020125614, ISSN: 1748-605X, DOI: 10.1088/1748-6041/2/2/008
- CHIARA COMPAGNIN ET AL: "The cellular uptake of meta-tetra(hydroxyphenyl)chlorin entrapped in organically modified silica nanoparticles is mediated by serum proteins", NANOTECHNOLOGY, IOP, BRISTOL, GB, vol. 20, no. 34, 26 August 2009 (2009-08-26), pages 345101, XP020160823, ISSN: 0957-4484
- KONAN-KOUAKOU Y N ET AL: "In vitro and in vivo activities of verteporfin-loaded nanoparticles", JOURNAL OF CONTROLLED RELEASE, ELSEVIER, AMSTERDAM, NL, vol. 103, no. 1, 2 March 2005 (2005-03-02), pages 83 - 91, XP027664436, ISSN: 0168-3659, [retrieved on 20050302]
- BOURDON O ET AL: "A comparative study of the cellular uptake, localization and phototoxicity of meta-tetra(hydroxyphenyl) chlorin encapsulated in surface-modified submicronic oil/water carriers in HT29 tumor cells", JOURNAL OF PHOTOCHEMISTRY AND PHOTOBIOLOGY B: BIOLOGY, ELSEVIER SCIENCE S.A., BASEL, CH, vol. 55, no. 2-3, 1 April 2000 (2000-04-01), pages 164 - 171, XP002522470, ISSN: 1011-1344, DOI: 10.1016/S1011-1344(00)00043-9
- KRUEGER THORSTEN ET AL: "Experimental photodynamic therapy for malignant pleural mesothelioma with pegylated mTHPC.", LASERS IN SURGERY AND MEDICINE 2003, vol. 32, no. 1, 2003, pages 61 - 68, ISSN: 0196-8092
- SEHGAL INDER ET AL: "Photoinduced cytotoxicity and biodistribution of prostate cancer cell-targeted porphyrins", JOURNAL OF MEDICINAL CHEMISTRY, AMERICAN CHEMICAL SOCIETY, US, vol. 51, no. 19, 9 October 2008 (2008-10-09), pages 6014 - 6020, XP009137593, ISSN: 0022-2623, DOI: 10.1021/JM800444C

## Description

### Background of the Invention

### 1. Field of the invention

The present invention concerns the preparation of nanoparticle formulations containing hydrophobic photosensitizers and their use in photodynamic therapy, particularly for photodynamic tumor therapy, using intravenous administration.

### 2. Information Disclosure Statement

Photodynamic therapy (PDT) is one of the most promising new techniques now being explored for use in a variety of medical applications and particularly is a wellrecognized treatment for the destruction of tumors. Photodynamic therapy uses light and a photosensitizer (a dye) to achieve its desired medical effect. A large number of naturally occurring and synthetic dyes have been evaluated as potential photosensitizers for photodynamic therapy. Perhaps the most widely studied class of photosensitizers is the tetrapyrrolic macrocyclic compounds. Among them, especially porphyrins and chlorins have been tested for their PDT efficacy.

Porphyrins are macrocyclic compounds with bridges of one carbon atom joining pyrroles to form a characteristic tetrapyrrole ring structure. There are many different classes of porphyrin derivatives including chlorins containing one dihydro-pyrrole unit and bacteriochlorins containing two dihydro-pyrrole units. Both mentioned porphyrin derivatives possessing potential for PDT can either be derived from natural sources or from total synthesis.

Compared to porphyrins, chlorins have the advantage that they possess a more favorable absorption spectrum, i.e. they have a more intense absorption in the red and near-infrared region of the electromagnetic spectrum. As light of longer wavelength penetrates deeper into the tissue it is thus possible to treat e.g. more expanded tumors, when PDT is employed for tumor therapy.

Nevertheless, the use of PDT for the treatment of various types of disease has been limited due to the inherent features of photosensitizers (PS). These include their high cost, extended retention in the host organism, substantial skin phototoxicity, low solubility in physiological solutions (which also reduces their usefulness for intravascular administration as it can provoke thromboembolic accidents), and low targeting effectiveness. These disadvantages, particularly of PS in the prior art, had led to the administration of very high doses of a photosensitizer, which dramatically increase the possibility of accumulation of the photosensitizer in non-damaged tissues and the accompanying risk of affecting non-damaged sites upon irradiation.

Efforts to reduce cost and to decrease background toxicity have been underway but are unrelated to the developments of the present invention. Work to improve solubility in physiological solutions, effects of skin phototoxicity, retention in host organism and to a lesser extent targeting effectiveness are the areas where the present invention provides new and non-obvious improvements on the use of PDT to treat various neoplasia, hyperplasia and related diseases.

Most substances successfully employed for photodynamic tumor therapy are lipophilic substances, which due to their inherent low solubility in water need to be formulated in a proper way. Therefore, there is a great need for new formulations of tetrapyrrole-based photosensitizers to enhance their uptake in the body and their bioavailability.

Nanoparticles are intensively investigated as carriers for lipophilic drug substances. In fact, a nanoparticle formulation of the anti-cancer drug Paclitaxel based on human serum albumin (HSA) has been approved recently by regulatory authorities in Europe and the USA.

Nanoparticles in general are solid colloidal particles, typically, ranging in size from 10nm to 1000nm. They consist of macromolecular materials in which the active ingredient is dissolved, entrapped or encapsulated, and/or to which the active principle is absorbed or attached. Many different sorts of nanoparticle material have been investigated, i.e. quantum dots, silica-based nanoparticles, photonic crystals, liposomes, nanoparticles based on different polymers of natural and synthetic origin, and metal-based nanoparticles.

Nanoparticles in combination with photosensitizers have been investigated i.e. for many applications including imaging approaches, such as the nanoparticles, disclosed in Patent Publication N° US 2007/0148074A1 by Sadoqi et al., comprising biodegradable polymer materials entrapping near-infrared dyes for using them in bio-imaging. Additionally, other nanoparticle systems combining fluorescence imaging and magnetic resonance imaging, especially in combination with metal (iron) based nanoparticles are known in the art (see Mulder et. al, Nanomed., 2007, 2, 307-324; Kim et.al, Nanotechnol., 2002, 13, 610-614; Primo et al, J. Magnetism Magn. Mater., 2007, 311, 354-357) but such developments are unrelated to the present invention. Also, other nanoparticle formulations based on liposomes, quantum dots, inorganic materials (including metals) which are known in the art do not interfere with the present invention.

Most interesting as carrier systems for photosensitizers are nanoparticles that consist of biocompatible materials. Such carrier systems could significantly improve the treatment regimen of photodynamic therapy. A carrier system with such known high biocompatibility is e.g. poly(DL-lactic-co-glycolic acid) (PLGA). PLGA material has successfully been formulated as nanoparticles.

There are a few examples of PLGA-based nanoparticles as carriers for photosensitizers known in the art (see Gomes et al., Photomed. Laser Surg., 2007, 25, 428-435; Ricci-Junior et al., J. Microencapsul., 2006, 23, 523-538; Ricci-Junior et al., Int. J. Pharm., 2006, 310, 187-195; Saxena et al., Int. J. Pharm., 2006, 308, 200-204; McCarthy et al., Abstracts of Papers, 229th ACS Meeting, 2005; Vargas et al., Int. J. Pharm., 2004, 286, 131-145; Konan et al., Eur. J. Pharm. Sci., 2003, 18, 241-249; Konan et al., Eur. J. Pharm. Biopharm., 2003, 55, 115-124; Vargas et al., Eur. J. Pharm. Biopharm., 2008, 69, 43-53; Pegaz et al., J. Photochem. Photobiol. B: Biology, 2005, 80, 19-27).

Nevertheless, some of the known art mentioned above concentrates on other types of photosensitizers such as the invention disclosed in Patent Application N° WO97010811A1 comprising the photosensitizers Zinc(II) phthalocyanine and indocyanine green which are unrelated to the present invention.

In other cases, such as in Patent Publication N° WO03097096A1 and patent, US7,455,858 B2 by Allemann et al., the PLGA-based nanoparticles used as carriers for photosensitizers, are intended for a rapid release of the drug, preferably within about 60 seconds, after the nanoparticles are introduced into an environment containing serum proteins and, therefore, are not well suited for a drug transport to the target cells and tissues. There is a lack of targeting effectiveness of the PLGA-based nanoparticles as the photosensitizer is released within seconds after being introduced into an environment containing serum proteins. Furthermore, for the preparation of small sized and monodisperse PLA- or PLGA-based nanoparticles known in art high concentrations of polyvinyl alcohol (PVA) stabilizer in the range of about 5-20% in the aqueous phase are employed.

The application of a nanoparticle formulation for parenteral administration in clinical practice requires that the sterility of the formulation according to pharmacopoeial specifications can be assured. The problem of sterility of nanoparticle photosensitizer formulations involving PLGA is challenging because of the lability of the nanoparticle matrix material as well as the lability of the photosensitizer. Conventional methods of sterilization (autoclaving, use of ethylene oxide, gamma-irradiation) are incompatible with these photosensitizer formulations (see Athanasiou et. al, Biomaterials, 1996, 17, 93-102; Volland et. al, J. Contr. Rel., 1994, 31, 293-305). An alternative is the sterile filtration through membrane filters of a defined size for such chemically and thermally sensitive materials. Pore size for sterile filtration is usually 0.22µm whereas nanoparticles of the present invention are in the size range between 100 and 500nm. Therefore, sterile filtration has its drawbacks and is not generally compatible with the nanoparticles that are subject of the present invention.

Also, for a clinical application it is highly desirable that the formulation can be freeze dried and later be reconstituted in an aqueous medium. In particular, it is difficult to develop sterile nanoparticle formulations and nanoparticle formulations suitable for freeze drying in the case of photosensitizers of the present invention which are of the chlorin or bacteriochlorin type (i.e. tetrapyrroles carrying one or two dihydro-pyrrole units), because such systems are especially sensitive to oxidation and photo-chemical modifications induced by the handling conditions that are often used for nanoparticle preparation (see Hongying et al., Dyes Pigm., 1999, 43, 109-117; Hadjur et al., J. Photochem. Photobiol. B: Biology, 1998, 45, 170-178; Bonnett et al., J. Chem. Soc. Perkin Trans. 2, 1999, 325-328). These photosensitizers of the chlorin or bacteriochlorin type which possess one or two dihydro-pyrrole units, respectively, differ significantly in their chemical and physical behaviour from the corresponding porphyrins (see Bonnett et al., J. Chem. Soc. Perkin Trans. 2, 1999, 325-328; Bonnett et al., J. Porphyrins Phthalocyanines, 2001, 5, 652-661). The second point, that the problem of sterility and freeze-drying has up to now been addressed only for chemically more tetrapyrrole-based photosensitizers, holds especially for the green porphyrins described by Allemann et al. or for the photosensitizers investigated by Konan et al.

Konan, Y. N. et al. in Photochemistry and Photobiology 2003, 77(6), 638-644 studied the encapsulation of porphyrin into nanoparticles, in particular the cellular uptake, the subcellular localization and the effect of serum on the photodynamic activity.

Compagnin, C. in Nanotechnology 2009, 20, 345101, studied the cellular uptake of mTHPC entrapped in organically modified silica nanoparticles and found that the cellular uptake is mediated by serum proteins.

Konan-Kouakou, Y.N. et al. Journal of Controlled Release 2005, 103, 83-91 deals with in vitro and in vivo activities of verteporfin-loaded nanoparticles.

Bourdon, O. et al. J. Photochem. Photobiol. B Biol. 2000, 55, 164 -171 present a comparative study of the cellular uptake, localization and phototoxicity of mTHPC encapsulated in suface-modified submircron oil/water carriers in HT29 tumor cells.

The PLGA-based nanoparticles used as carriers for photosensitizers known in the art either do not address such problems as sterility and freeze-drying or if so, the investigated photosensitizers are less problematic in this respect because of their more stable chemical structure.

This is the case of Patent Publication N° WO 2006/133271 A2 which discloses Photosensitizer Nanoparticle Aptamer Conjugates comprising a photosensitizer that forms the central core of the nanoparticle, a biodegradable polymer shell and a targeting aptamer (e.g. ErbB3 receptor-specific aptamer) but does not address the problem of sterility of the nanoparticle photosensitizer formulations nor the freeze-drying process required to obtain a stable nanoparticle photosensitizer formulation.

In spite of the already mentioned drawbacks, present invention provides PLGA-based nanoparticle formulations and methods of preparation for photosensitizers suitable for parenteral application that can be prepared for such sensitive compounds as chlorins and bacteriochlorins.

There remain these problems in the art for which the present invention addresses and provides solutions.

### Objectives and Brief Summary of the Invention

It is an objective of the present invention to provide nanoparticle formulations for hydrophobic photosensitizers used for photodynamic therapy based on biocompatible PLGA material.

It is another objective of the present invention to provide nanoparticle formulations for hydrophobic photosensitizers of the tetrapyrrole type, namely chlorins and bacteriochlorins, based on poly(DL-lactic-co-glycolic acid) (PLGA) and the stabilizing agent poly(vinyl alcohol).

It is yet another objective of the present invention to provide nanoparticle formulations for hydrophobic photosensitizers which enable a high variation of photosensitizer loading efficiency (2 to 320µg photosensitizer per mg nanoparticles) to the particle system giving the opportunity of a high variability in drug pharmacokinetics.

It is a further objective of the present invention to provide nanoparticle formulations for hydrophobic photosensitizers which enable an effective drug transport to target cells and tissues combined with a drug release after cellular accumulation

It is yet a further objective of the present invention to provide methods for the production of sterile PLGA-based photosensitizer-loaded nanoparticles of a mean particle size less than 500nm, with the photosensitizers being chlorins or bacteriochlorins. The nanoparticles of the present invention are stable enough to allow freeze drying and reconstitution in an aqueous medium.

It is yet another object of the present invention to provide methods for the use of nanoparticle photosensitizer formulations based on PLGA in PDT for the treatment of tumors and other neoplastic diseases, dermatological disorders, ophthalmological disorders, urological disorders, arthritis and similar inflammatory diseases.

These objects are achieved by a formulation and a method having the features of the independent claim. Advantageous embodiments and refinements are specified in claims dependent thereon.

Briefly stated, present invention provides compositions, which are stable in storage, and a method of production of pharmaceutical based nanoparticulate formulations for clinical use in photodynamic therapy comprising a hydrophobic photosensitizer, poly(lactic-co-glycolic) acid and the stabilizing agent PVA.

These nanoparticulate formulations provide therapeutically effective amounts of photosensitizer for parenteral administration. In particular, tetrapyrrole derivatives can be used as photosensitizers whose efficacy and safety are enhanced by such nanoparticulate formulations. It also teaches the method of preparing PLGA-based nanoparticles under sterile conditions. In one of the preferred embodiments of the present invention PLGA-based nanoparticles have a mean particle size less than 500nm and the photosensitizer is temoporfin, 5,10,15,20-tetrakis(3-hydroxyphenyl)-chlorin (*m*THPC). In another embodiment, the photosensitizer 2,3-dihydroxy-5,10,15,20-tetrakis(3-hydroxyphenyl)-chlorin (*m*THPD-OH) is formulated as a nanoparticle for parenteral administration. The formulations can be used for treating hyperplasic and neoplasic conditions, inflammatory problems, and more specifically to target tumor cells.

The above, and other objects, features and advantages of the present invention will become apparent from the following description read in conjunction with the accompanying figures.

Examples relating to nanoparticle formulations comprising mTHPP are outside the scope of the present invention.

### Brief Description of the Figures

Fig. 1 depicts the preferred structures of chlorins and bacteriochlorins to be used in present invention.
Fig. 2 depicts the structure of the specifically preferred chlorins to be formulated in nanoparticles according to the present invention.
Fig. 3 shows a curve which indicates that depending upon the ratio of drug to PLGA a drug loading efficiency between 2 and 320µg *mTHPP* per milligram PLGA could be achieved.
Fig. 4A and B shows confocal laser scanning microscopy images of cellular uptake and intracellular distribution of PLGA based nanoparticles with the photosensitizer 5,10,15,20-tetrakis(3-hydroxyphenyl)-porphyrin (*m*THPP).
Fig. 5A and B shows confocal laser scanning microscopy images of cellular uptake and intracellular distribution of PLGA based nanoparticles with the photosensitizer 5,10,15,20-tetrakis(3-hydroxyphenyl)-chlorin (*m*THPC).
Fig. 6 shows the results of phototoxicity of 3 µM *m*THPC, and different *m*THPC loaded PLGA nanoparticles on Jurkat cells after different incubation times.
Fig. 7 shows the results of intracellular uptake of 3 µM *m*THPC, and different *m*THPC loaded PLGA nanoparticles by Jurkat cells after different incubation times.

### Detailed Description of Preferred Embodiments

The methods of preparation of the described nanoparticle systems of the present invention provide systems that enable a drug release over several hours even in the presence of serum proteins and, therefore, are suitable for a drug transport to target cells and tissues. This is in contrast with the immediate decomposition of particles and release of photosensitizers in the prior art use of PLGA nanoparticle systems. Moreover, a high variability of drug release kinetics is obtained depending on the way the excipients are used during particle preparation.

As outlined above, questions such as sterility and freeze drying are vital to the development of nanoparticle formulations of photosensitizers. It has now been found that such PLGA-based nanoparticle photosensitizer formulations suitable for clinical applications can be prepared by an aseptic manufacturing process. Thus, present invention provides methods for the production of sterile PLGA-based photosensitizer-loaded nanoparticles of a mean particle size less than 500nm, with the photosensitizers being chlorins or bacteriochlorins. Additionally, nanoparticle pharmaceutical formulations of the present invention are stable enough to allow freeze drying and reconstitution in an aqueous medium. Therefore present invention addresses the problem of suitable nanoparticle pharmaceutical formulations of hydrophobic photosensitizers for photodynamic therapy that meet the necessities for a parenteral administration in clinical practice.

Therapeutic uses of nanoparticle photosensitizer formulations based on PLGA in PDT include dermatological disorders, ophthalmological disorders, urological disorders, arthritis and similar inflammatory diseases. More preferably, therapeutic uses of nanoparticle photosensitizer formulations based on PLGA in PDT comprise the treatment of tumor tissues, neoplasia, hyperplasia and related conditions.

The described nanoparticle systems of PLGA-based nanoparticle formulations for chlorins and bacteriochlorins for parenteral application can be prepared in the presence of reduced amounts of stabilizers (i.e. 1.0% PVA). The systems enable a drug release over several hours even in the presence of serum proteins and, therefore, are suitable for transporting a drug to target cells and tissues. The prolonged drug release enables the attachment of drug targeting ligands to the particle surface (such as antibodies) for a more advanced transport of photosensitizer to target cells and tissues.

The present invention is based in part upon the surprising discovery that during particle preparation excipients such as polyvinyl alcohol (PVA) can be used in a way, so that 1) the photosensitizer is attached by incorporation in the particle matrix, 2) is attached by adsorption to the particle matrix, 3) or is attached by incorporation in and adsorption to the particle matrix, resulting in a high variability of drug release kinetics.

In a specifically preferred embodiment of the present invention the PLGA-based nanoparticles have a mean particle size less than 500nm and the photosensitizer is temoporfin, 5,10,15,20-tetrakis(3-hydroxyphenyl)-chlorin *(mTHPC).*

In another embodiment of the present invention the PLGA-based nanoparticles have a mean particle size less than 500nm and the photosensitizer is 2,3-dihydroxy-5,10,15,20-tetrakis(3-hydroxyphenyl)-chlorin (*m*THPD-OH).

The invention provides methods to prepare formulations of photosensitizercontaining nanoparticles preferably using photosensitizers of the chlorin and bacteriochlorin type. The nanoparticles prepared by the methods disclosed below have a predictable size and uniformity (in size distribution). The nanoparticles are prepared in an aseptic manufacturing process. Preferred PLGA-based nanoparticles have a mean size less than 500nm. The term "diameter" is not intended to mean that the nanoparticles have necessarily a spherical shape. The term refers to the approximate average width of the nanoparticles.

In a preferred embodiment of the present invention the PLGA-based nanoparticles can be prepared so that the photosensitizer loading can be varied in a wide concentration range (2 to 320µg photosensitizer per mg nanoparticles).

In a specifically preferred embodiment of the present invention the PLGA-based nanoparticles can be prepared so that the photosensitizer is attached by incorporation in the particle matrix, is attached by adsorption to the particle matrix or is attached by incorporation in and adsorption to the particle matrix, resulting in a high variability of drug release kinetics.

Drug targeting effectiveness of present nanoparticle systems may be enhanced with one or more ligands bound to PLGA-nanoparticles, maintaining the photosensitizer chemical entity by not bonding to photosensitizer molecules.

The nanoparticles of the invention may be dehydrated for improved stability on storage. The preferred method of dehydration is freeze-drying or lyophilisation. Optionally, a lyoprotectant may be used as an additive to improve the stability during the freeze-drying and during reconstitution in an aqueous medium.

In another embodiment, the present invention provides methods for the use of nanoparticle photosensitizer formulations based on PLGA in PDT, comprising the administration of the nanoparticles, their accumulation in the target tissue and the activation of the photosensitizer by light of a specific wavelength. The administration is preferably by parenteral means such as intravenous injection.

### Materials used for the preparation of the photosensitizer-loaded nanoparticles

### Polymer

The polymer to be used in the present invention is poly(D,L-lactide-co-glycolide) PLGA, preferably characterised by a copolymer ratio of 50:50 or 75:25. PLGA to be used for the preparations underlying the present invention was obtained from Boehringer Ingelheim (Resomer RG502H and Resomer RG504H).

### Photosensitizers

The photosensitizers to be used in the present invention are tetrapyrroles of the chlorin and bacteriochlorin type. Such photosensitizers can either be derived from natural sources or by total synthesis. The total synthesis of chlorins and bacteriochlorins can be performed by first synthesizing the porphyrin and then transferring it to a chlorine or bacteriochlorin system (e.g. R. Bonnett, R. D. White, U.-J. Winfield, M. C. Berenbaum, Hydroporphyrins of the meso-tetra(hydroxyphenyl)porphyrin series as tumor photosensitizers, Biochem. J. 1989, 261, 277-280).

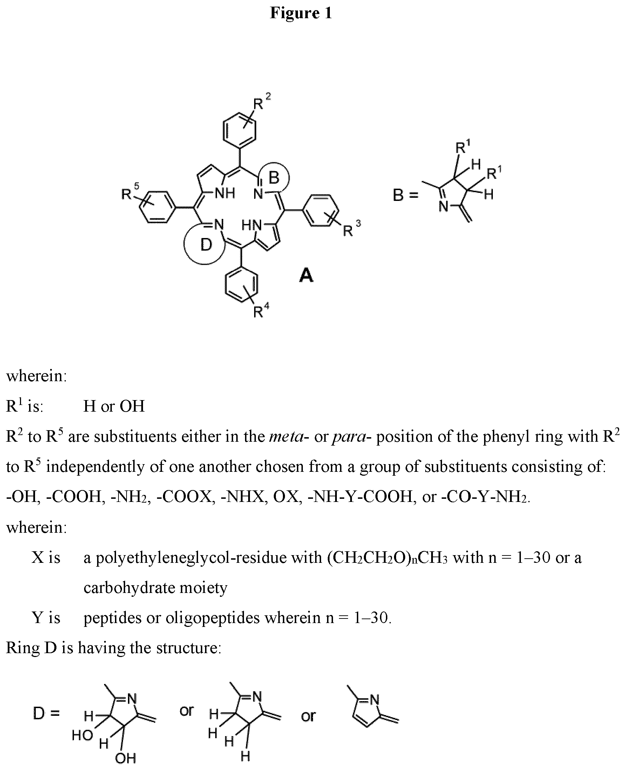

The chlorins and bacteriochlorins to be used with the present invention have the structure depicted in Fig. 1.

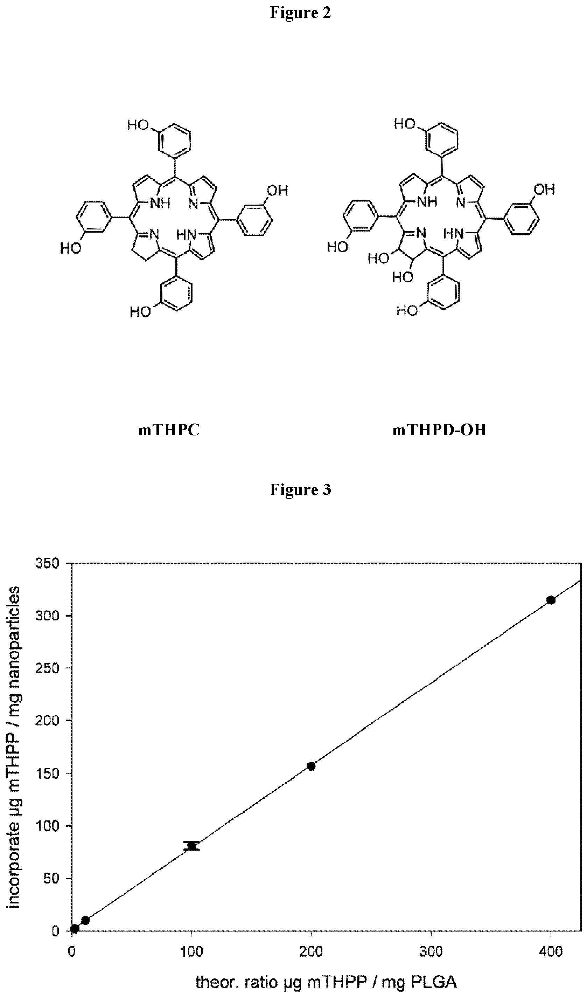

Specifically preferred chlorins to be formulated in nanoparticles according to the present invention have the structure depicted in Fig. 2.

The PLGA-based nanoparticles of the present invention were prepared by an emulsion-diffusion-evaporation process using an Ultra-Turrax dispersion unit. An adsorptive binding of the photosensitizer to the particle matrix, an incorporative binding into the particle matrix and a combination of adsorptive and incorporative binding to the particle matrix can be achieved. Drug loaded nanoparticles can be freeze dried in the presence of cryoprotective agents such as glucose, trehalose, sucrose, sorbitol, mannitol and the like.

The present invention is further illustrated by the following examples.

Examples relating to nanoparticle formulations comprising mTHPP are outside the scope of the present invention.

### EXAMPLE 1a

Preparation and characterization of PLGA-based nanoparticles with the photosensitizer 5,10,15,20-tetrakis(3-hydroxyphenyl)-porphyrin (*m*THPP); combination of adsorptive and incorporative binding to the particle matrix

The PLGA-based nanoparticles were prepared by an emulsion-diffusion-evaporation process using an Ultra-Turrax dispersion unit (Ultra Turrax T25 digital, IKA, Staufen, Germany).

An amount of 500mg PLGA (Resomer RG 502H or 504H) was dissolved in 5mL ethyl acetate (Fluka, Steinheim, Germany). To this solution different amounts of mTHPP were added. Quantities in the range of 1 to 200mg were under evaluation. Commonly, 50mg of *mTHPP* were used.

This organic solution was added to 10mL of a 1% polyvinyl alcohol (PVA) stabilized aqueous solution. With an Ultra-Turrax dispersion unit (17,000rpm, 5min) an oil-in-water nanoemulsion was formed. After this preparing step the emulsion was added to 40mL of an aqueous PVA stabilized solution to induce the formation of nanoparticles after complete diffusion of the organic solvents into the aqueous external phase. Permanent mechanical stirring (550rpm) was maintained for 18h to allow the complete evaporation of ethyl acetate.

The particles were purified by 5 cycles of centrifugation (16,100G; 8min) and redispersion in 1.0mL water in an ultrasonic bath (5min).

All of the aqueous solutions used for particle preparation were sterile and prefiltered through a membrane with a pore size of 0.22µm (Schleicher und Schüll, Dassel, Germany). All of the equipment used was autoclaved at 121°C over 20min. All handling steps for particle preparation were performed under a laminar airflow cabinet.

Average particle size and polydispersity were measured by photon correlation spectroscopy using Zetasizer 3000 HS_{A} (Malvern Instruments, Malvern, UK). Nanoparticle content was determined by microgravimetry.

*Direct quantification procedure:* The PLGA-nanoparticles were dissolved in acetone and the solution was measured photometrically at 512nm for *mTHPP* to determine the content of photosensitizer. Depending upon the ratio of drug to PLGA a drug loading efficiency between 2 and 320µg *mTHPP* per milligram PLGA could be achieved (Fig. 3).

*Lyophilisation of the nanoparticles can be performed according to the following protocol:* For the freeze drying process trehalose was added at a concentration of 3% (m/V) to the nanoparticle samples. The samples were transferred to a freeze drier and the shelf temperature was reduced from 5°C to -40°C at a rate of 1°C/min. The pressure was 0.08mbar. These parameters were held for 6h. By increasing the temperature from -40°C to -25°C at 0.5°C/min the primary drying was achieved. The pressure remained unchanged.

At the end of the primary drying heat ramp, a Pressure Rise Test (PRT) was performed. With termination of the primary drying the secondary drying followed by increasing the temperature at a rate of 0.2°C/min to 25°C. This temperature was held for 6h at a pressure of 60mT (=0.08mbar).

### EXAMPLE 1b

Preparation and characterization of PLGA-based nanoparticles with the photosensitizer 5,10,15,20-tetrakis(3-hydroxyphenyl)-chlorin *m*THPC; combination of adsorptive and incorporative binding to the particle matrix
Nanoparticles were prepared according to example 1a with the exception that *m*THPC was used instead of *m*THPP. *m*THPC was photometrically quantified at 517nm. Depending upon the ratio of drug to PLGA a drug loading efficiency between 2 and 320µg *m*THPC per milligram PLGA could be achieved.

*m*THPC loaded nanoparticles were characterized as described within example 1a.

### EXAMPLE 1c

Preparation and characterization of PLGA-based nanoparticles with the photosensitizer 5,10,15,20-tetrakis(3-hydroxyphenyl)-porphyrin (*m*THPP); solely adsorptive binding to the particle matrix

The above described standard method (example 1a) was used to prepare empty PLGA-nanoparticles. The preparation steps were performed as described for example 1a except for the addition of the photosensitizer mTHPP.

In the next step a PVA-stabilized *m*THPP solution was prepared. Therefore, 25mg *m*THPP was solved in 5mL ethyl acetate and afterwards 10mL of a 1% aqueous PVA solution was added. With an Ultra-Turrax dispersion unit an emulsion was prepared. The emulsion was added to 40mL PVA solution (1%). Permanent mechanical stirring (550rpm) was maintained for 18h to allow the complete evaporation of ethyl acetate.

A volume of the PLGA-nanoparticle suspension corresponding to 10mg nanoparticles was centrifuged (16,100G; 8min) and the supernatant was discarded. The nanoparticles were redispersed in the PVA-stabilized *m*THPP solution using an ultrasonic bath (5min).

The mixture was agitated (Thermomixer comfort, Eppendorf, Hamburg, Germany) for 18h (500rpm, 20°C) to achieve adsorption equilibrium of *m*THPP to the particle surface. The nanoparticles were purified as previously described.

Depending upon the ratio of drug to PLGA a drug loading efficiency between 2 and 80µg *m*THPP (according to the standard protocol typically 20µg) per milligram PLGA could be achieved.

*m*THPP loaded (adsorbed) nanoparticles were characterized and lyophilized as described within example 1a.

### EXAMPLE 1d

Preparation and characterization of PLGA-based nanoparticles with the photosensitizer 5,10, 15,20-tetrakis(3-hydroxyphenyl)-chlorin *m*THPC; solely adsorptive binding to the particle matrix

Nanoparticles were prepared according to example 1c with the exception that *m*THPC was used instead of *m*THPP. *m*THPC was photometrically quantified at 517nm.

Depending upon the ratio of drug to PLGA a drug loading efficiency between 2 and 80µg *m*THPC (according to the standard protocol typically 20µg) per milligram PLGA could be achieved.

*m*THPC loaded (adsorbed) nanoparticles were characterized and lyophilized as described within example 1a.

### EXAMPLE 1e

Preparation and characterization of PLGA-based nanoparticles with the photosensitizer 5,10,15,20-tetrakis(3-hydroxyphenyl)-porphyrin (*m*THPP); solely incorporative binding to the particle matrix

PLGA nanoparticles were prepared according to example 1a. The resulting nanoparticles were washed with aqueous 5% (m/V) PVA solution instead of purified water in order to displace the adsorptive bound *m*THPP from the nanoparticle surface. After 3 cycles of washing with PVA solution the nanoparticles were further purified by repeated centrifugation and redispersion in purified water.

Depending upon the ratio of drug to PLGA a drug loading efficiency between 15 and 80µg *m*THPP (according to the standard protocol typically 50µg) per milligram PLGA could be achieved.

*m*THPP loaded (incorporated) nanoparticles were characterized and lyophilized as described within example 1a.

### EXAMPLE 1f

Preparation and characterization of PLGA-based nanoparticles with the photosensitizer 5,10,15,20-tetrakis(3-hydroxyphenyl)-chlorin *m*THPC; solely incorporative binding to the particle matrix.

Nanoparticles were prepared according to example 1e with the exception that *m*THPC was used instead of *m*THPP. *m*THPC was photometrically quantified at 517nm.

Depending upon the ratio of drug to PLGA a drug loading efficiency between 15 and 80µg *m*THPC (according to the standard protocol typically 50µg) per milligram PLGA could be achieved.

*m*THPC loaded (incorporated) nanoparticles were characterized and lyophilized as described within example 1a.

### EXAMPLE 2a

Cell uptake and cell adhesion, respectively, of PLGA-based nanoparticles with the photosensitizer 5,10,15,20-tetrakis(3-hydroxyphenyl)-porphyrin (*m*THPP)
To show the cellular uptake and cell adhesion, respectively, and the intracellular distribution of the PLGA-based nanoparticles, the confocal laser scanning microscopy was used. HT29 cells were cultured on glass slides (BD Biosciences GmbH, Heidelberg) and incubated with the nanoparticulate formulation for 4h at 37°C. Following, the cells were washed twice with PBS and the membranes were stained with Concanavalin A AlexaFluor350 (50µg/ml; Invitrogen, Karlsruhe) for 2 min. Cells were fixed with 0.4 % paraformaldehyde for 6 min. After fixation, the cells were washed two times and embedded in Vectashield HardSet Mounting Medium (Axxora, Grünberg). The microscopy analysis was performed with an Axiovert 200 M microscope with a 510 NLO Meta device (Zeiss, Jena), a chameleon femtosecond or an argon ion laser and the LSM Image Examiner software. The green fluorescence of the PLGA based nanoparticles leading from incorporated Lumogen Yellow^{(R)} (BASF; Ludwigshafen) and the red autofluorescence of the photosensitizer 5,10,10,20-tetrakis(3-hydroxyphenyl)-porphyrin (*m*THPP) was used to determine the distribution.

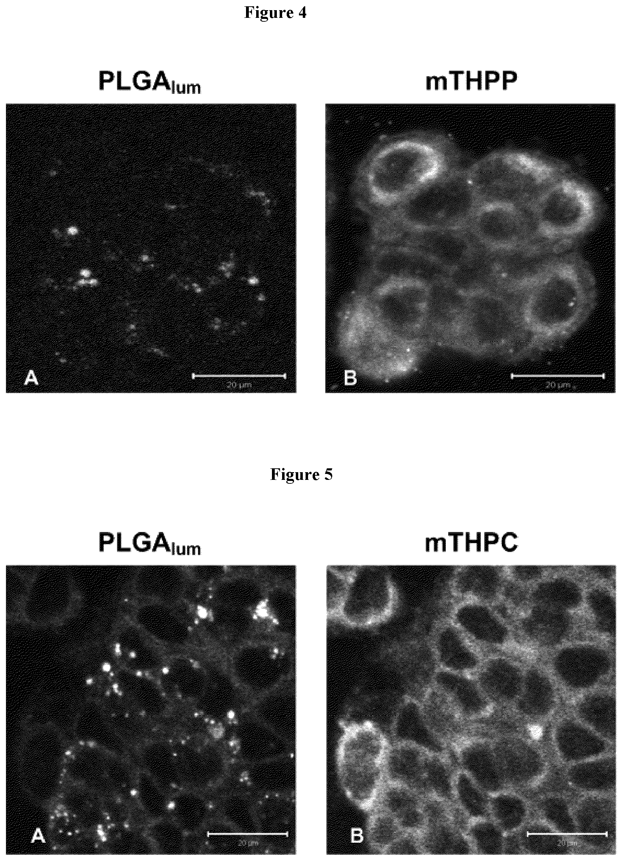

Figures 4A and B shows the cellular uptake/adhesion and intracellular distribution of PLGA based nanoparticles with the photosensitizer 5,10,15,20-tetrakis(3-hydroxyphenyl)-porphyrin (*m*THPP) studied by confocal laser scanning microscopy. HT29 cells were cultured on glass slides and incubated with the nanoparticles for 4 h at 37°C. The red autofluorescence of the photosensitizer mTHPP was used. The nanoparticles contain incorporated Lumogen Yellow^{(R)} (green).
(Fig. 4A) displays the green nanoparticles channel; (Fig. 4B) displays the red photosensitizer channel. Scale bar = 20°µm.

### EXAMPLE 2b

Cell uptake and cell adhesion, respectively, of PLGA-based nanoparticles with the photosensitizer 5,10,15,20-tetrakis(3-hydroxyphenyl)-chlorin *(mTHPC).*

Figures 5A and B shows the cellular uptake/adhesion and intracellular distribution of PLGA based nanoparticles with the photosensitizer 5,10,10,20-tetrakis(3-hydroxyphenyl)-chlorin (*m*THPC) studied by confocal laser scanning microscopy. HT29 cells were cultured on glass slides and incubated with the nanoparticles for 4 h at 37°C. The red autofluorescence of the photosensitizer mTHPC was used. The nanoparticles contain incorporated Lumogen Yellow^{(R)} (green).
(Fig. 5A) displays the green nanoparticle channel; (Fig. 5B) displays the red photosensitizer channel. Scale bar = 20 µm.

### EXAMPLE 3

Intracellular uptake and photodynamic activity of *m*THPC photosensitizer-loaded PLGA nanoparticles
The samples listed in Table 1 were tested for intracellular uptake and phototoxicity of *m*THPC-PLGA-nanoparticles.

| Table 1. Samples tested for intracellular uptake and phototoxicity of mTHPC-PLGA-Nanoparticles | | |
|---|---|---|
| 1 | *m*THPC-incorporated + adsorbed | 81.46µg/mg NP |
| 2 | *m*THPC-incorporated | 23.52µg/mg NP |
| 3 | *m*THPC-adsorbed | 19.27µg/mg NP |

All cell samples were incubated with a dye concentration of 3µM *m*THPC in the medium (RPMI1640) for 1h, 3h, 5h, 24h in Jurkat cell suspensions

Phototoxicity of different *m*THPC loaded PLGA nanoparticles on Jurkat cells after different incubation times was assessed with the Trypan blue test and apoptotic change of the cell shape. Experiments were performed with a 660nm LED light source, an exposure time of 120s and a light dose of 290mJ/cm².

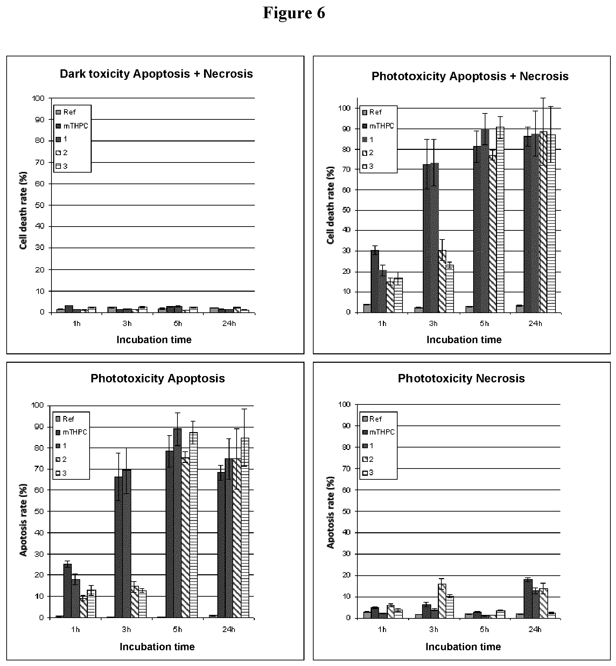

Figure 6 shows the results of phototoxicity of 3 µM *m*THPC, and different *m*THPC loaded PLGA nanoparticles on Jurkat cells after different incubation times. Left: Rate of apoptosis, Right: Rate of necrosis. (reference cells were incubated and irradiated without photosensitizer). Light source: LED λ_{exc}= 660nm; Exposure time: 120s; Light dose: 290mJ/cm². The experiments were repeated twice and for each measurement the cell number was counted three times two hours after light exposure to get an average. Error bars represent the standard deviation of six measurements (n=6).

Experiments to quantify the intracellular uptake of different *m*THPC loaded PLGA nanoparticles were also performed.

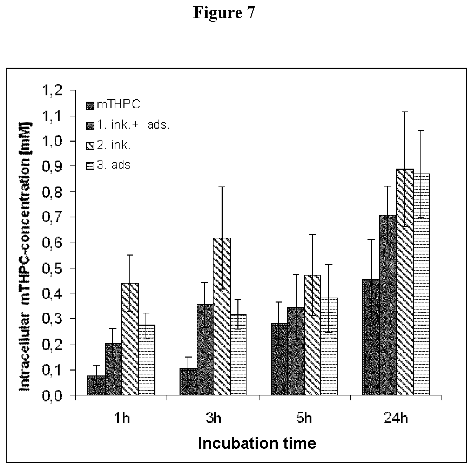

Figure 7 shows the results of intracellular uptake of 3 µM *m*THPC, and different *m*THPC loaded PLGA nanoparticles by Jurkat cells after different incubation times. The experiments were repeated twice and for each measurement the cell number was counted three times to get an average. Error bars represent the standard deviation of six measurements (n=6).

After incubation the cells were counted using a haemocytometer, washed (PBS, 400g, 3min, 2x) and the cell pellet was stored frozen overnight at -20°C to disrupt the cell membranes.

From these cells the *mTHPC* was extracted in ethanol using ultrasound.

The *m*THPC concentration in the ethanol extract was determined via fluorescence using a standard fluorescence series. For the calculation of intracellular concentration the diameter of the cells was assumed to be 10µm (3 measurements).

All three PLGA-nanoparticles transport *m*THPC into the cells. The transport into the cells occurs in a faster way, when the *m*THPC is incorporated in the NPs.

After 5h incubation all NPs cause a high phototoxicity.

## Claims

1. A nanoparticle pharmaceutical formulation for photodynamic therapy comprising:
- poly(DL-lactic-co-glycolic) acid (PLGA) particles having a mean size of less than 500nm in diameter;
- a therapeutically effective amount of a tetrapyrrole-based hydrophobic photosensitizer;
- a stabilizing agent;
wherein said photosensitizer is a chlorin or bacteriochlorin derivative according to formula A 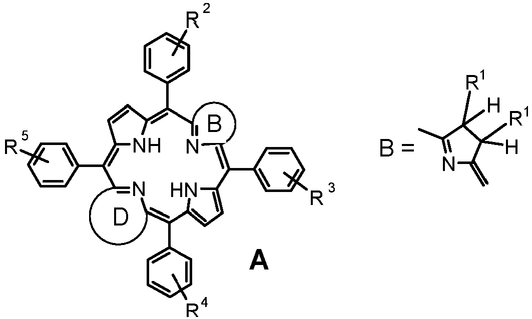
wherein:
R¹ is H or OH
R² to R⁵ are substituents either in the *meta-* or *para-* position of the phenyl ring with R² to R⁵ independently of one another chosen from a group of substituents consisting of: -OH, -
COOH, -NH₂, -COOX, -NHX, OX, -NH-Y-COOH, or -CO-Y-NH₂.
wherein:
X is a polyethyleneglycol-residue with (CH₂CH₂O)ₙCH₃ with n = 1-30 or a carbohydrate moiety;
Y is peptides or oligopeptides wherein n = 1-30;
ring D is having the structure: 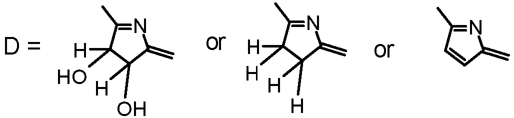
wherein said stabilizing agent is poly(vinyl alcohol) (PVA).

2. The nanoparticle pharmaceutical formulation for photodynamic therapy according to claim 1, wherein the therapeutically effective concentration of the photosensitizer is highly variable from 10 to 320µg per mg nanoparticle.

3. The nanoparticle pharmaceutical formulation for photodynamic therapy according to any of claims 1 or 2,
wherein said photosensitizer is selected from the group consisting of temoporfin (mTHPC), and 2,3-dihydroxy-5,10,15,20-tetrakis(3-hydroxyphenyl)-chlorin (mTHPD-OH).

4. The nanoparticle pharmaceutical formulation for photodynamic therapy according to any of claims 1 to 3,
wherein said drug loaded nanoparticles is freeze dried in the presence of cryoprotective agents selected from the group of glucose, trehalose, sucrose, sorbitol, mannitol and combinations of them.

5. The nanoparticle pharmaceutical formulation for photodynamic therapy according to any of claims 1 to 4,
wherein said formulation comprises drug targeting ligands attached to the nanoparticle surface for a transport of photosensitizer to target cells and tissues.

6. A method of preparation of nanoparticle pharmaceutical formulation according to any of claims 1 to 5, comprising the steps of:
a. disolving PLGA in an organic solvent;
b. filtering said PLGA solution through a filtration unit;
c. adding the photosensitizer through adsorptive binding on particle surface, incorporative binding or combination of both;
d. filtering a stabilizing aqueous solution including PVA through a filtration unit and adding said stabilizing aqueous solution to form an oil-in-water nanoemulsion; and
e. purifying the resulting nanoparticles.

7. The method of preparation according to claim 6,
wherein an organic solvent is ethyl acetate.

8. The nanoparticle pharmaceutical formulation according to any of claims 1 to 5,
for use in the photodynamic therapy of tumors and other neoplastic diseases.

9. The nanoparticle pharmaceutical formulation according to any of claims 1 to 5,
for use in the photodynamic therapy of dermatological disorders, ophthalmological disorders or urological disorders.

10. The nanoparticle pharmaceutical formulation according to any of claims 1 to 5,
for use in the photodynamic therapy of arthritis and inflammatory diseases.

11. The nanoparticle pharmaceutical formulation for photodynamic therapy according to any of claims 8 to 10,
wherein said formulation is administered by parenteral means including intravenous injection.

## Patentansprüche

1. Pharmazeutische Nanopartikelformulierung für die photodynamische Therapie mit:
- Poly(DL-lactid-co-glykolid)säure-Partikel (PLGA) mit einer mittleren Größe von weniger als 500 nm im Durchmesser;
- einer therapeutisch wirksamen Menge eines hydrophoben Photosensibilisators auf Tetrapyrrolbasis;
- einem Stabilisierungsmittel;
wobei der Photosensibilisator ein Chlorin- oder Bakteriochlorinderivat gemäß der Formel A ist 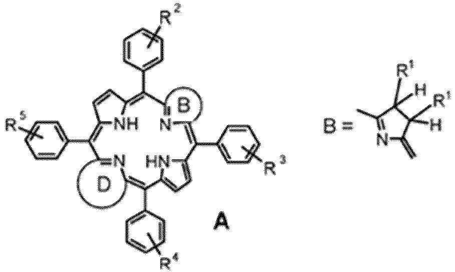
wobei:
R¹ ist H oder OH
R² bis R⁵ sind Substituenten entweder in der *meta-* oder *para*-Position des Phenylrings,
wobei R² bis R⁵ unabhängig voneinander ausgewählt sind aus einer Gruppe von Substituenten, bestehend aus: -OH, -COOH, -NH₂, -COOX, -NHX, OX, -NH-Y-COOH
oder -CO-Y-NH₂.
wobei:
X ist ein Polyethylenglykol-Rest mit (CH₂CH₂O)ₙCH₃ mit n = 1-30 oder ein Kohlenhydratanteil;
Y ist Peptide oder Oligopeptide, wobei n = 1-30;
Ring D hat die Struktur: 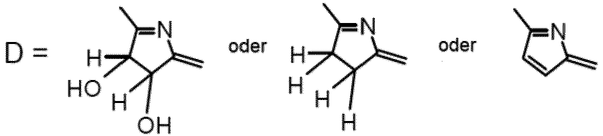
wobei das Stabilisierungsmittel Poly(vinylalkohol) (PVA) ist.

2. Pharmazeutische Nanopartikelformulierung für die photodynamische Therapie nach Anspruch 1, wobei die therapeutisch wirksame Konzentration des Photosensibilisators hochvariabel zwischen 10 bis 320µg pro mg Nanopartikel liegt.

3. Pharmazeutische Nanopartikelformulierung für die photodynamische Therapie nach einem der Ansprüche 1 oder 2,
wobei der Photosensibilisator ausgewählt ist aus der Gruppe bestehend aus Temoporfin (mTHPC) und 2,3-Dihydroxy-5,10,15,20-tetrakis(3-hydroxyphenyl)-chlorin (mTHPD-OH).

4. Pharmazeutische Nanopartikelformulierung für die photodynamische Therapie nach einem der Ansprüche 1 bis 3,
wobei die arzneimittelbeladenen Nanopartikel in Gegenwart von Kälteschutzmitteln, ausgewählt aus der Gruppe von Glucose, Trehalose, Saccharose, Sorbit, Mannit und Kombinationen davon, gefriergetrocknet sind.

5. Pharmazeutische Nanopartikelformulierung für die photodynamische Therapie nach einem der Ansprüche 1 bis 4,
wobei die Formulierung Liganden für den Wirkstofftransport umfasst, die an die Oberfläche der Nanopartikel gebunden sind, um den Photosensibilisator zu den Zielzellen und -geweben zu transportieren.

6. Verfahren zur Herstellung einer pharmazeutischen Nanopartikelformulierung nach einem der Ansprüche 1 bis 5 mit den folgenden Schritten:
a. Auflösen von PLGA in einem organischen Lösungsmittel;
b. Filtrieren der PLGA-Lösung durch eine Filtrationseinheit;
c. Hinzufügen des Photosensibilisators durch adsorptive Bindung an die Partikeloberfläche, inkorporative Bindung oder eine Kombination aus beidem;
d. Filtrieren einer stabilisierenden wässrigen Lösung, die PVA enthält, durch eine Filtrationseinheit und Hinzufügen der stabilisierenden wässrigen Lösung, um eine Öl-in-Wasser-Nanoemulsion zu bilden; und
e. Reinigung der resultierenden Nanopartikel.

7. Verfahren zur Herstellung nach Anspruch 6,
wobei ein organisches Lösungsmittel Ethylacetat ist.

8. Pharmazeutische Nanopartikelformulierung nach einem der Ansprüche 1 bis 5,
zur Verwendung bei der photodynamischen Therapie von Tumoren und anderen neoplastischen Erkrankungen.

9. Pharmazeutische Nanopartikelformulierung nach einem der Ansprüche 1 bis 5,
zur Verwendung bei der photodynamischen Therapie von dermatologischen, ophthalmologischen oder urologischen Erkrankungen.

10. Pharmazeutische Nanopartikelformulierung nach einem der Ansprüche 1 bis 5,
zur Verwendung bei der photodynamischen Therapie von Arthritis und entzündlichen Erkrankungen.

11. Pharmazeutische Nanopartikelformulierung für die photodynamische Therapie nach einem der Ansprüche 8 bis 10,
wobei die Formulierung durch parenterale Mittel einschließlich intravenöser Injektion verabreicht wird.

## Revendications

1. Formulation pharmaceutique nanoparticulaire pour thérapie photodynamique, comprenant :
- des particules de poly(acide DL-lactique-co-acide glycolique) (PLGA) ayant une taille moyenne de moins de 500 nm en diamètre ;
- une quantité thérapeutiquement efficace d'un photosensibilisateur hydrophobe à base de tétrapyrrole ;
- un agent stabilisant ;
ledit photosensibilisant étant un dérivé de chlorine ou bactériochlorine selon la formule A 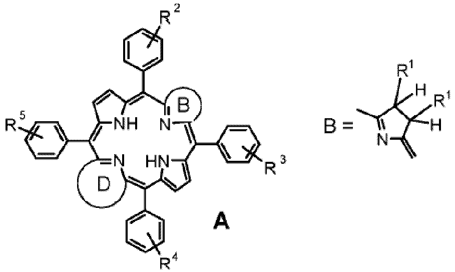
dans laquelle :
R¹ est H ou OH
R² à R⁵ sont des substituants en position soit *méta* soit *para* du cycle phényle, R² à R⁵ étant choisis indépendamment les uns des autres dans un groupe de substituants constitué par : -OH, -COOH, -NH₂, -COOX, -NHX, OX, -NH-Y-COOH, ou -CO-Y-NH_{2,},
où
X est un résidu de polyéthylèneglycol comportant (CH₂CH₂O)ₙCH₃ où n = 1-30 ou un fragment glucidique ;
Y est des peptides ou oligopeptides dans lesquels n = 1-30 ;
le cycle D a la structure : 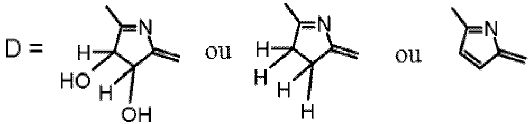
ledit agent stabilisant étant le poly(alcool vinylique) (PVA),

2. Formulation pharmaceutique nanoparticulaire pour thérapie photodynamique selon la revendication 1, dans laquelle la concentration thérapeutiquement efficace du photosensibilisateur est hautement variable de 10 à 320 µg par mg de nanoparticule.

3. Formulation pharmaceutique nanoparticulaire pour thérapie photodynamique selon l'une quelconque des revendications 1 et 2,
dans laquelle ledit photosensibilisateur est choisi dans le groupe constitué par la témoporfine (mTHPC), et la 2,3-dihydroxy-5,10,15,20-tétrakis(3-hydroxyphényl)-chlorine (mTHPD-OH).

4. Formulation pharmaceutique nanoparticulaire pour thérapie photodynamique selon l'une quelconque des revendications 1 à 3,
dans laquelle lesdites nanoparticules chargées de médicament sont lyophilisées en présence d'agents cryoprotecteurs choisis dans le groupe constitué par le glucose, le tréhalose, le saccharose, le sorbitol, le mannitol et des associations de ceux-ci.

5. Formulation pharmaceutique nanoparticulaire pour thérapie photodynamique selon l'une quelconque des revendications 1 à 4,
dans laquelle ladite formulation comprend des ligands vecteurs de médicament fixés à la surface de la nanoparticule pour un transport de photosensibilisateur vers des cellules et tissus cibles.

6. Procédé de préparation de formulation pharmaceutique nanoparticulaire selon l'une quelconque des revendications 1 à 5, comprenant les étapes consistant à :
a. dissoudre du PLGA dans un solvant organique ;
b. filtrer ladite solution de PLGA à travers une unité de filtration ;
c. ajouter le photosensibilisateur par liaison par adsorption à la surface des particules, liaison par incorporation ou combinaison des deux ;
d. filtrer une solution aqueuse stabilisante incluant du PVA à travers une unité de filtration et ajouter ladite solution aqueuse stabilisante pour former une nanoémulsion huile-dans-eau ; et
e. purifier les nanoparticules résultantes.

7. Procédé de préparation selon la revendication 6,
dans lequel un solvant organique est l'acétate d'éthyle.

8. Formulation pharmaceutique nanoparticulaire selon l'une quelconque des revendications 1 à 5,
pour utilisation dans la thérapie photodynamique de tumeurs et d'autres maladies néoplasiques.

9. Formulation pharmaceutique nanoparticulaire selon l'une quelconque des revendications 1 à 5,
pour utilisation dans la thérapie photodynamique de troubles dermatologiques, troubles ophtalmologiques ou troubles urologiques

10. Formulation pharmaceutique nanoparticulaire selon l'une quelconque des revendications 1 à 5,
pour utilisation dans la thérapie photodynamique de l'arthrite et de maladies inflammatoires.

11. Formulation pharmaceutique nanoparticulaire pour thérapie photodynamique selon l'une quelconque des revendications 8 à 10,
dans laquelle ladite formulation est administrée par voie parentérale incluant injection intraveineuse.
